# EUROPÄISCHE PATENTANMELDUNG

(11) **EP 1 820 520 A1**
(43) Veröffentlichungstag der Anmeldung: **22.08.2007**
(21) Anmeldenummer: 06101315.7
(22) Anmeldetag: 06.02.2006
(51) Int. Cl.: A61L 15/26, B32B 5/32, B32B 27/06, B32B 27/40, B29C 47/02, B29C 47/88

(54) **Wundabdeckung und dessen Extrusionsbeschichtungsherstellungsverfahren**

(71) Anmelder: Collano AG, 6203 Sempach-Station (CH)
(72) Erfinder: Leumann, Manuel, 5712 Beinwil am See (CH); Dobmann, Andreas, 6208 Oberkirch (CH)
(74) Vertreter: Hepp, Dieter

(57) **Zusammenfassung**

Die Erfindung betrifft ein Herstellungsverfahren für einen Schaumstoff-Wundverband mit einer aussenliegenden Folienschicht, sowie einen solchermassen erhaltenen Wundverband. Bei dem Verfahren gemäss der Erfindung wird die Folienschicht mittels Extrusion direkt auf der Schaumstoffunterlage erzeugt. Es resultiert ein zuverlässigerer Verbund zwischen Schaumstoffschicht und Folie, ohne dass hierdurch die Aufnahmefähigkeit des Schaumstoffs für Wundexsudat beeinträchtigt würde. Das vorgestellte Verfahren ist einfach, kostengünstig, und alle Anforderungen aus dem Medizinalbereich (z.B. Lösungsmittelfreiheit) können erfüllt werden.

## Beschreibung

Die Erfindung betrifft das Gebiet der auf Schaumstoffmaterialien basierenden Wundabdeckungen, insbesondere Herstellungsverfahren für solche Wundabdeckungen.

In der Wundversorgung finden heute vermehrt Schaumstoff-basierte Wundabdeckungen Verwendung, insbesondere für chronische Wunden. Derartige Schaumstoff-Wundabdeckungen haben den Vorteil, dass Wundexsudat zuverlässig von der Wunde entfernt werden kann aufgrund der Saugfähigkeit des Schaumstoffs. Gegenüber altbekannnten Wundabdeckungen wie bspw. Gaze, Mullbinden etc. bieten Schaumstoff-basierte Wundabeckungen also den Vorteil einer grösseren Aufnahmekapazität für Wundexsudat, sowie den weiteren Vorteil, dass kein Verkleben mit der Wunde eintritt und somit ein schmerzfreier Verbandwechsel möglich ist.

Auf der wundabgewandten Seite sind diese Schaumstoff-Wundabdeckungen in der Regel mit einer Folie abgedeckt. Diese Folie stellt einerseits einen Schutz gegen eindringende Bakterien dar und regelt darüber hinaus auch den Gasaustausch mit der Umgebung. Über eine gezielte Einstellung der Wasserdampfdurchlässigkeit dieser Folie kann sichergestellt werden, dass unterhalb der Wundabdeckung ein genügend feuchtes Klima herrscht, ohne dass es zu einer Mazerisation der Haut kommt. Zudem schützt diese Folie Kleidung und sonstiges Verbandsmaterial vor austretendem Wundexsudat.

Derzeit werden solche Folien mit der Schaumstoffunterlage verklebt, indem entweder lösungsmittelbasierte Klebstoffe aufgebracht (typischerweise aufgesprüht) oder auch Heiss-Schmelzklebstoffe aufgetragen werden. Der Klebstoffauftrag kann hierbei entweder einseitig (typischerweise auf die Folie) oder beidseitig, also auf die Folie und den Schaum, erfolgen. Alternativ kann bspw. auch ein wärmeaktivierbares Klebevlies zwischen Folie und Schaumstoff eingelegt werden, wodurch der Verbund bewirkt wird. Es muss jedoch unter Anwendung eines Klebstoffs stets eine Zwischenschicht erzeugt werden, um die Verbindung zwischen Schaumstoff und Folie sicherzustellen.

Kritisch ist hierbei stets, dass die Klebeschicht den Transport von Wasserdampf nicht behindern darf; in der Regel sollte die Durchlässigkeit über 1000 g/m²/Tag liegen. Eine solche Durchlässigkeit kann bspw. über ein offenes Beschichtungsbild mit Klebstoff oder durch einen vollflächig aufgetragenen, jedoch wasserdampfdurchlässigen Klebstofffilm erreicht werden.

Die Gewährleistung der ausreichenden Wasserdampfdurchlässigkeit durch die Klebstoffschicht stellt in der Praxis während der Herstellung ein nicht zu unterschätzendes Problem dar, während die Durchlässigkeit durch die Folie und den Schaumstoff mit einfachen Routineversuchen ermittelt und eingestellt werden kann. Zudem verteuert die notwendige Klebstoffschicht die Herstellung insgesamt. Zudem ist die Verbindung von Folie und Schaumstoff durch eine Klebstoffschicht in der Praxis oftmals nicht überzeugend, da es immer wieder zu Ablösungen der Folie von dem Schaumstoff kommt.

Aus US 5,147,338 ist es bekannt, bei einer Wundabdeckung einen Polyurethan-Film auf einen Polyurethan-Schaumstoff aufzusprühen. Hierdurch kann die o.g. Klebstoffschicht entfallen. Das Aufsprühen eines Polyurethanfilms auf den Schaumstoff hat jedoch eine Vielzahl von Nachteilen: Erstens kann ein gleichmässiger Auftrag nur schwer gewährleistet werden. Zweitens penetriert das aufgesprühte Polyurethanmaterial signifikant in den Schaumstoff, was die Aufnahmefähigkeit des Schaumstoffs für Wundexsudat erheblich senken kann. Zudem müssen, um die Versprühbarkeit von Polyurethanmaterialien zu gewährleisten, Lösungsmittel zugesetzt werden; Lösungsmittelreste in der Wundabdeckung können jedoch nicht toleriert werden.

Es ist daher eine Aufgabe der vorliegenden Erfindung, die Nachteile des bekannten zu vermeiden, insbesondere ein eine Schaumstoff-Wundabdeckung und ein Herstellungsverfahren für eine Schaumstoff-Wundabdeckung bereitzustellen, welches in der Durchführung einfach und kostengünstig ist, keine Rückstände (z.B. Lösungsmittelreste) im fertigen Produkt enthält bzw hinterlässt und im Ergebnis einen hervorragenden und dauerbelastbaren Verbund zwischen Schaumstoff und Folie sicherstellt, ohne dass die Aufnahmefähigkeit des Schaumstoffs für Wundexsudat herabgesetzt wird.

Diese Aufgabe wird durch eine Wundabdeckung und ein Verfahren zur Herstellung einer solchen Wundabdeckung gelöst, wie in den Ansprüchen definiert.

Das erfindungsgemässe Verfahren zur Herstellung eines mindestens zweischichtigen flächigen Artikels, insbesondere einer Wundabdeckung, umfassend eine erste Schicht aus einem Schaumstoffmaterial, und eine zweite Schicht aus einem Folienmaterial, welche zweite Schicht unmittelbar an eine Hauptfläche der ersten Schicht angrenzt, umfasst die folgenden Schritte:
(a) Bereitstellen des Schaumstoffmaterials der ersten Schicht;
(b) Extrudieren mindestens eines Materials auf eine Hauptfläche des Schaumstoffmaterials, welches Material anschliessend zur zweiten Schicht verfestigt.

Überraschenderweise hat sich herausgestellt, dass sich durch das Extrudieren des Folienmaterials direkt (also ohne Klebstoff-Zwischenschicht) auf das Schaumstoffmaterial Wundabdeckungen herstellen lassen, welche sowohl eine hervorragende Festigkeit des Verbunds als auch nur eine minimale Penetration des Folienmaterials in den Schaumstoff aufweisen. Die Aufnahmefähigkeit des Schaumstoffs ist daher nicht negativ beeinflusst, wie es hingegen bei aufgesprühtem Folienmaterial beobachtet wird. Das Folienmaterial muss für die Extrusion (im Gegensatz zur Sprühtechnik) nicht gelöst vorliegen, wodurch keinerlei Rückstände von Lösungsmitteln in dem fertigen Produkt auftreten. Zudem ist die Herstellung einfach zu handhaben, die Schichtdicke des Auftrags kann mit herkömmlichen Extrusionsanlagen (insbesondere mit gängigen Breitschlitzdüsen) zuverlässig eingestellt und konstant gehalten werden.
(Hauptfläche bedeutet dabei diejenige Oberfläche der Schaumstoff-Schicht, die durch das Folienmaterial abgedeckt werden soll.)

In weiteren bevorzugten Ausführungsformen der Erfindung wird als Schaumstoffmaterial ein Polyurethan-Schaumstoff bereitgestellt. Vorzugsweise ist das bereitgestellte Schaumstoffmaterial hydrophil. Besonders bevorzugt ist das Schaumstoffmaterial im wesentlichen offenzellig. Vorzugsweise kann eine durchschnittliche Porengrösse im Bereich von 0,02mm bis 0,2mm gewählt werden. Geeignete und besonders bevorzugte hydrophile, polyurethanbasierte Schaumstoffmaterialien sind dem Fachmann bekannt und im Markt erhältlich (bspw. Typ Vivo MCF 03 von Corpura B.V., 4879 NE Etten-Leur, The Netherlands; oder Typ 3014 von Polymer Health Technology, Ebbw Vale, NP23 8XE, United Kingdom).

In einer weiteren vorteilhaften Ausgestaltung der Erfindung wird das Schaumstoffmaterial in einer Dicke zwischen 1mm bis 10mm, vorzugsweise zwischen 3mm und 5mm, bereitgestellt. Derartige Dicken haben sich als hervorragender Kompromiss zwischen erforderlicher Aufnahmefähigkeit für Wundexsudat und Handhabbarkeit der Wundabdeckung erwiesen.

In besonders vorteilhaften Ausgestaltungen der Erfindung wird das mindestens eine Material in Schritt b) derart auf das Schaumstoffmaterial extrudiert, dass sich eine zweite Schicht mit einer Dicke zwischen 15µm bis 100µm, vorzugsweise 20µm bis 40µm, ausbildet. Dicken in den angegebenen Bereichen beeinflussen die Handhabbarkeit der Wundabdeckung wenig bis gar nicht, und das o.g. Steuern der Wasserdampfdurchlässigkeit der resultierenden Folie kann in diesem Dickenbereich an alle in der Praxis auftretenden Anforderungen angepasst werden.

Es wurde als besonders vorteilhaft gefunden, dass die Extrusion des mindestens einen Materials in Schritt b) bei einer Temperatur im Bereich zwischen 180°C und 220°C, insbesondere zwischen 200°C und 210°C erfolgt.

In einer weiteren, besonders bevorzugten Ausführungsform können in Schritt b) zwei Schichten erzeugt werden, insbesondere durch Extrusion zweier Materialien, entweder sequentiell oder mittels Co-Extrusion. Auch das Aufbringen einer ersten Folienschicht mittels Extrusion und einer weiteren Schicht bspw. mittels Sprühen ist möglich. Insbesondere ist es durch die Erfindung ermöglicht, bspw. eine dünne, dem Schaumstoff zugewandte Schicht zu erzeugen, welche optimiert ist für die Haftung auf dem Schaumstoff; Schichtdicken von 5µm bis 10µm haben sich hierfür bereits als ausreichend erwiesen. Anschliessend kann als aussenliegende, zweite Schicht noch eine Schicht mit z.B. höherer mechanischer Festigkeit (typischerweise in einer Dicke von etwa 10µm bis 20µm) aufgetragen, oder gleichzeitig coextrudiert werden. Auf diese Weise können also Folienschichten erzeugt werden, welche aus mindestens zwei Teil-Schichten aufgebaut sind, wobei die Folienschicht insgesamt sehr dünn sein kann, jedoch trotzdem eine sehr gute Haftung auf dem Schaumstoffmaterial und ein weiches Griffbild aufweist. Zudem ist es durch die Verwendung zweier unterschiedlicher Folienschichten auch möglich, die Wasserdampfdurchlässigkeit über die Materialauswahl der zusätzlichen Schicht zu steuern, unabhängig von der Gesamtdicke der Folienschicht.

In einer weiteren Ausführungsform der Erfindung kann das mindestens eine Material während der Extrusion in Schritt b) aufgeschäumt werden. Die Aufschäumung eines Materials während der Extrusion ist dem Fachmann geläufig und kann mit konventionellen Mitteln (bspw. durch den Zusatz eines Treibmittels wie Azodicarbonamid) erfolgen. Vorteilhafterweise könnte bspw. eine erste, aufgeschäumte Folienschicht auf den Schaumstoff extrudiert werden, mit einer geringeren Porengrösse als die darunter liegende Schaumstoffschicht. Anschliessend (oder auch gleichzeitig durch Coextrusion) kann dann die abschliessende Folienschicht aufgebracht werden. Durch die somit nicht abrupte, sondern stufenweise Abnahme der Porosität kann eine weitere Verbesserung der Haftung zwischen wundzugewandtem Schaumstoff und aussenliegender Folie erzielt werden, wobei zudem noch eine üblicherweise gewünschte, optisch glattere wundabgewandte Seite resultiert.

Besonders bevorzugt wird nach der Extrusion des Materials in Schritt b) das Extrudat mittels einer gekühlten Walze an die erste Schicht bzw. den Schaumstoff angepresst. Hierbei findet jedoch keine bzw. nur eine minimale Penetration des Schaumstoffs statt, weil das Extrudat vorgeformt auf den Schaumstoff auftrifft und insbesondere die Viskosität der Schmelze bereits möglichst hoch ist.

Es ist weiter bevorzugt, dass in Schritt b) ein thermoplastisches Polyurethanmaterial, insbesondere ein Polyetherpolyurethan, bereitgestellt wird. Thermoplastische Polyetherpolyurethane mit Zulassung für den medizinischen Bereich sind dem Fachmann bekannt und im Markt erhältlich. Besonders geeignete thermoplastische Materialien weisen gemäss ISO 1133 einen Melt Flow Index (MFI) zwischen 5 und 50 g/10min bei 170°C und einem Stempelgewicht von 21.6kg auf.

Ein weiterer Aspekt der Erfindung betrifft also einen zweischichtigen flächigen Artikel, umfassend eine erste Schicht aus einem Schaumstoffmaterial, und eine zweite Schicht aus einem Folienmaterial, welche zweite Schicht unmittelbar an eine Hauptfläche der ersten Schicht angrenzt, insbesondere eine Wundabdeckung, wobei bei einer Porengrösse des im wesentlichen offenzelligen Schaumstoffmaterials im Bereich von 0,02mm bis 0,2mm das Material der zweiten Schicht nicht mehr als 0,01mm in das Schaumstoffmaterial eingedrungen ist. Eine derartige minimale Eindringtiefe lässt sich insbesondere durch ein Verfahren wie vorstehend im Detail dargestellt bewirken, wohingegen bei konventionellem Sprühauftrag von Polyurethan-Lösungen eine Eindringtiefe von mindestens etwa 0,05mm resultiert.

Ein zusätzlicher Aspekt der Erfindung betrifft die Verwendung eines Extrusionsverfahrens in der Herstellung einer Folienschicht direkt auf einer Schaumstoff-Wundabdeckung.

Die Erfindung wird nachfolgend anhand von Ausführungsbeispielen und Figuren erläutert, ohne dass der Gegenstand der Erfindung auf diese Ausführungsbeispiele zu beschränken wäre. Es zeigen:
- Fig. 1:: Wundabdeckung mit Schaumstoffschicht und aussenliegender Folie;

- Fig. 2:: Wundabdeckung mit Schaumstoffschicht und zwei aussenliegenden Folienschichten;
- Fig. 3:: Herstellungsverfahren, schematisch.

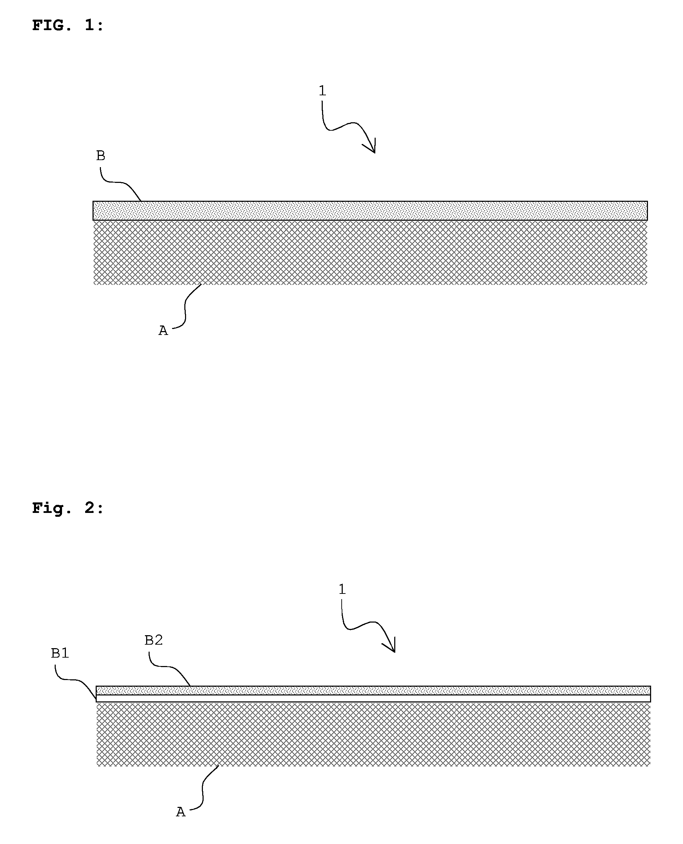

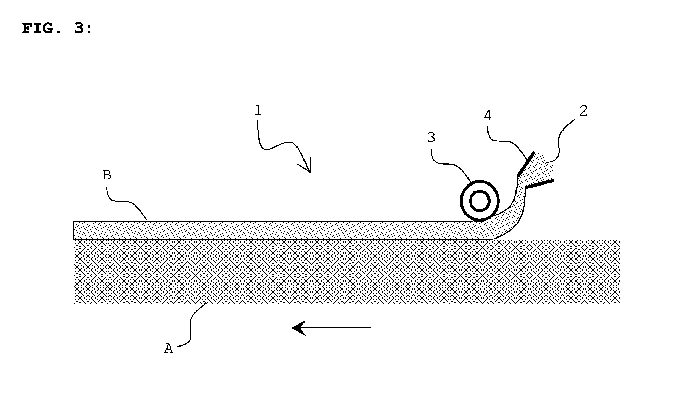

Fig. 1 zeigt schematisch vereinfacht eine erfindungsgemässe Wundabdeckung 1, erhältlich mit dem vorstehend im Detail beschriebenen und in Fig. 3 illustrierten Verfahren, umfassend eine der Wunde zugewandte erste Schicht A aus einem Schaumstoffmaterial, und eine zweite Schicht B aus einem Folienmaterial. Die beiden Schichten A und B sind ohne eine Zwischenschicht verbunden; insbesondere liegt keine Klebstoffschicht zwischen der ersten Schicht A und der zweiten Schicht B. Besonders zuverlässige Verbindungen werden erhalten, wenn die Materialien von erster Schicht A und zweiter Schicht B chemisch verwandt sind. Vorteilhaft handelt es sich bei der ersten Schicht A um einen hydrophilen Polyurethanschaumstoff, und bei Schicht B um eine Schicht aus Polyetherpolyurethan; aufgrund der ähnlichen chemischen Natur der beiden Schichten lässt sich eine besonders stabile Verbindung erzielen.

Fig. 2 zeigt eine Ausführungsform einer erfindungsgemässen Wundabdeckung, bei welcher auf einer ersten Schicht A aus einem Schaumstoffmaterial eine zweite Schicht aufgetragen ist, welche die Teilschichten B1 und B2 umfasst. Die Schichten B1 und B2 können entweder gemeinsam, bspw. mittels Coextrusion (oder sequentieller Extrusion) erzeugt werden. Auch das Erzeugen nur der Schicht B1 mittels Extrusion ist möglich, wobei die Schicht B2 z.B. anschliessend aufgesprüht wird. Die Schicht B2 kann, jedoch muss nicht flächig aufgetragen sein; insbesondere kann es sich hierbei auch um einen Aufdruck handeln, umfassend Schriftzeichen wie Herstellerangaben, Markennamen oder auch Schnittlinien für die Erleichterung eines passgenauen Zuschnitts der Wundabdeckung vor Ort.

Fig. 3 illustriert schematisch eine Ausführungsform des erfindungsgemässen Herstellungsverfahrens anhand einer Wundabdeckung. Eine erste Schicht A eines Schaumstoffmaterials wird in einer herkömmlichen, geeigneten Extrusionsanlage (nicht im Detail gezeigt) entlang der Pfeilrichtung gefördert. Um eine Förderung des Schaumstoffmaterials zuverlässig zu gewährleisten, kann ggf. ein steifes Trägermaterial für Schicht A vorgesehen werden. Über eine Düse 4, typischerweise eine Breitschlitzdüse, wird ein thermoplastisches Material (hier: ein thermoplastisches Polyetherpolyurethan) extrudiert, typischerweise bei einer Temperatur zwischen 180°C und 220°C. Die Düse 4 hat hierbei keinen direkten Kontakt mit der Schicht A, sondern dass Material 2 verlässt die Düse vorgeformt und wird gewissermassen auf der Schicht A abgelegt und anschliessend vorzugsweise mit einer gekühlten Walze 3 derart an die Schicht A angedrückt, dass eine feste Verbindung zwischen Schicht A und der aus dem Material 2 gebildeten Schicht B gebildet wird.

Mit dem erfindungsgemässen Verfahren erhaltene Wundverbände wurden auf die Festigkeit des Verbunds zwischen Folie und Schaumstoff analysiert und mit einem derzeit handelsüblichen Produkt verglichen. Als Material für die Herstellung der Folienschicht (Schicht B) wurde Perlathane^{®} D16N85 (Hersteller Merquinsa), MFI 10g/10min bei 170°C (Stempelgewicht 21.6kg), Extrusionstemperatur 205°C verwendet; die jeweils verwendete Schaumstoffunterlage (Schicht A) ist in Tabelle 1 angegeben. Hierbei wurden die folgenden Testmethoden verwendet:

### Testmethode 1: Haftung

Auf die Polyurethan-Schaumseite von 100 cm² (10x10 cm Muster) des Verbunds aus Schicht A und Schicht B werden 5ml einer wässrigen 0.9% NaCl-Lösung gegeben. Die befeuchteten Muster werden anschliessend bei 40°C in einer gesättigten Wasserdampfatmosphäre während 24 h gelagert. Nach der Entnahme der Prüflinge werden diese während 10 Minuten auf Raumtemperatur abgekühlt. Anschliessend wird die Haftung zwischen Polyurethan-Folie und Polyurethan-Schaum von Hand qualitativ beurteilt. Zum Vergleich werden Proben beurteilt, welche nicht feucht und warm gelagert wurden. Die gefundene Haftung wird nach einem Notensystem (1=unbrauchbar, 2=schwach, 3=ungenügend, 4=genügend, 5=gut, 6=ausgezeichnet) klassifiziert.

### Testmethode 2: Dichtigkeit

Auf die Polyurethan-Schaumseite von 100 cm² (10 x10 cm Muster) des Verbunds aus Schicht A und Schicht B werden 5 ml einer wässrigen 0.9% NaCl-Lösung, welche mit 0.1% Methylenblau eingefärbt ist, gegeben. Nach 1 Stunde (Raumtemperatur) wird die Dichtigkeit auf der Polyurethan-Folienseite (Schicht B) optisch beurteilt. Die gefundene Dichtigkeit wird nach einem Notensystem (1=unbrauchbar, 2=schwach, 3=ungenügend, 4=genügend, 5=gut, 6=ausgezeichnet) klassifiziert.

Die erhaltenen Resultate sind in Tabelle 1 angegeben:

**Tabelle 1: Beurteilung von Haftung und Dichtigkeit erfindungsgemässer Wundverbände.**

| PUR-Film extrudiert auf: | Vivo MCF 03 (Corpura B.V.) | Foam 3014 (Polymer Health Technology) | **Referenzmuster** 3M Foam Dressing (Art. 90601) |
|---|---|---|---|
| Haftung zwischen Schaum und Film (Blindwert) | 6 | 6 | 5 |
| Haftung zwischen Schaum und Film nach 24h Feuchtlagerung bei 40°C | 5 | 5 | 3-4 |
| Dichtigkeit nach 1 Stunde | 6 | 6 | 6 |

Es ist ersichtlich, dass die Festigkeit des Verbunds zwischen Schicht A (Schaumstoff) und Schicht B (Folie) bei den erfindungsgemässen Wundverbänden bzw. durch das erfindungsgemässe Herstellungsverfahren für einen solchen Wundverband unter typischer Praxisbeanspruchung verbessert ist.

## Patentansprüche

1. Verfahren zur Herstellung eines mindestens zweischichtigen flächigen Artikels, insbesondere einer Wundabdeckung (1), umfassend eine erste Schicht (A) aus einem Schaumstoffmaterial, und eine zweite Schicht (B) aus einem Folienmaterial, welche zweite Schicht (B) unmittelbar an eine Hauptfläche der ersten Schicht (A) angrenzt, umfassend die folgenden Schritte:
(a) Bereitstellen des Schaumstoffmaterials der ersten Schicht (A);
(b) Flächiges extrudieren mindestens eines Materials (2) auf die Hauptfläche des Schaumstoffmaterials (A), und verfestigen des Materials (2) zur zweiten Schicht (B).

2. Verfahren gemäss Anspruch 1,
**dadurch gekennzeichnet, dass** als Schaumstoffmaterial der ersten Schicht (A) ein Polyurethan-Schaumstoff bereitgestellt wird.

3. Verfahren gemäss einem der Ansprüche 1 oder 2,
**dadurch gekennzeichnet, dass** die erste Schicht (A) hydrophil ist.

4. Verfahren gemäss einem der Ansprüche 1 bis 3,
**dadurch gekennzeichnet, dass** das Schaumstoffmaterial für die erste Schicht (A) in einer Dicke zwischen 1mm bis 10mm, vorzugsweise zwischen 3mm bis 5mm bereitgestellt wird.

5. Verfahren gemäss einem der Ansprüche 1 bis 4,
**dadurch gekennzeichnet, dass** das Schaumstoffmaterial der ersten Schicht (A) offenzellig ist.

6. Verfahren gemäss einem der Ansprüche 1 bis 5,
**dadurch gekennzeichnet, dass** das mindestens eine Material (2) in Schritt b) derart auf das Schaumstoffmaterial extrudiert wird, dass sich eine zweite Schicht (B) mit einer Dicke zwischen 15µm bis 100 µm, vorzugsweise zwischen 20µm bis 40µm ausbildet.

7. Verfahren gemäss einem der Ansprüche 1 bis 6,
**dadurch gekennzeichnet, dass** die Extrusion des mindestens einen Materials (2) in Schritt b) bei einer Temperatur im Bereich zwischen 180°C und 220°C, insbesondere zwischen 200°C und 210°C erfolgt.

8. Verfahren gemäss einem der Ansprüche 1 bis 7,
**dadurch gekennzeichnet, dass** in Schritt b) zwei Schichten (B1, B2) erzeugt werden, insbesondere durch Extrusion zweier Materialien, entweder sequentiell oder mittels Co-Extrusion.

9. Verfahren gemäss einem der Ansprüche 1 bis 8,
**dadurch gekennzeichnet, dass** das mindestens eine Material (2) während der Extrusion in Schritt b) aufgeschäumt wird.

10. Verfahren gemäss einem der Ansprüche 1 bis 9,
**dadurch gekennzeichnet, dass** nach der Extrusion des Materials (2) in Schritt b) das Extrudat mittels einer gekühlten Walze (3) an die erste Schicht (A) angepresst wird.

11. Verfahren gemäss einem der Ansprüche 1 bis 10,
**dadurch gekennzeichnet, dass** als Material (2) in Schritt b) ein thermoplastisches Polyurethanmaterial, insbesondere ein Polyetherpolyurethan, bereitgestellt wird.

12. Mindestens zweischichtiger flächiger Artikel, umfassend eine erste Schicht (A) aus einem Schaumstoffmaterial, und eine zweite Schicht (B) aus einem Folienmaterial, welche zweite Schicht unmittelbar an eine Hauptfläche der ersten Schicht (A) angrenzt, insbesondere eine Wundabdeckung (1),
**dadurch gekennzeichnet, dass**, insbesondere bei einer Porengrösse des im wesentlichen offenzelligen Schaumstoffmaterials im Bereich von 0,02mm bis 0,2mm, das Material der zweiten Schicht (B) nicht mehr als 0,01mm in das Schaumstoffmaterial eingedrungen ist.

13. Flächiger Artikel insbesondere gemäss Anspruch 12, erhältlich durch ein Verfahren gemäss einem der Ansprüche 1 bis 11.

14. Verwendung eines Extrusionsverfahrens im Aufbringen einer Folienschicht auf die Schaumstoffschicht Schaumstoff-Wundabdeckung.
